# EUROPÄISCHE PATENTSCHRIFT

(11) **EP 2 358 333 B1**
(45) Veröffentlichungstag und Bekanntmachung des Hinweises auf die Patenterteilung: **17.08.2016**
(21) Anmeldenummer: 09768073.0
(22) Anmeldetag: 10.12.2009
(51) Int. Cl.: A61K 8/11, A61K 8/25, A61K 8/81, A61K 8/92, A61Q 5/10

(54) **BESCHICHTETE FÄRBEMITTEL**
COATED COLORING AGENTS
AGENTS DE COLORATION ENROBÉS

(30) Priorität: 19.12.2008 DE 102008063800
(43) Veröffentlichungstag der Anmeldung: 24.08.2011
(73) Patentinhaber: Henkel AG & Co. KGaA, 40589 Düsseldorf (DE)
(72) Erfinder: WELZ, Carolin, 22459 Hamburg (DE); MANNECK, Hartmut, 23860 Klein Wesenberg (DE); KLEEN, Astrid, 20457 Hamburg (DE); AKRAM, Mustafa, 22455 Hamburg (DE)
(86) Internationale Anmeldenummer: PCT/EP2009/066838
(87) Internationale Veröffentlichungsnummer: WO 2010/069855

(56) Entgegenhaltungen:
- EP-A1- 1 752 191
- WO-A1-89/06531
- WO-A1-2005/044208
- WO-A2-2009/013779

## Beschreibung

Die Erfindung betrifft Mittel zum Färben von keratinhaltigen Fasern, insbesondere menschlicher Haare, die umhüllte Oxidationsfarbstoffvorprodukte enthalten. Dadurch wird eine gleichmäßigere Färbung sichergestellt werden, die unabhängig davon ist, welche Strähnen zuerst oder zuletzt mit dem Färbeprodukt benetzt werden. Die Umhüllung der Oxidationsfarbstoffvorprodukte besteht dabei aus einem Verkapselungsmaterial aus Methacrylsäure-, Methacrylsäureester- oder Vinylacetat-homo- oder -copolymeren oder Schellack sowie mindestens einem Trennmittel. Insgesamt wird damit die Einwirkzeit zeitlich angeglichen und insgesamt verkürzt, was außerdem eine Reduzierung von Haarschäden durch zu lange Einwirkzeiten bewirkt. Ein weiterer Gegenstand der vorliegenden Erfindung betrifft ein Verfahren zur Herstellung von Färbemitteln für keratinische Fasern, enthaltend beschichtete Partikel.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. Zur modischen Farbgestaltung von Frisuren oder zur Kaschierung von ergrautem oder gar weißem Haar mit modischen oder natürlichen Farbtönen greift der Verbraucher zu farbverändernden Mitteln. Diese Mittel sollen neben der gewünschten Färbeleistung möglichst minimale Schädigungen auf dem Haar hervorrufen und vorzugsweise sogar zusätzliche Pflegeeigenschaften besitzen.

Zur Bereitstellung farbverändernder kosmetischer Mittel, insbesondere für die Haut oder keratinhaltige Fasern wie beispielsweise menschliche Haare, kennt der Fachmann je nach Anforderungen an die Färbung diverse Färbesysteme.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muss aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Als Entwicklerkomponenten werden üblicherweise primäre aromatische Amine mit einer weiteren, in para- oder ortho-Position befindlichen, freien oder substituierten Hydroxy- oder Aminogruppe, heterozyklische Hydrazone, Diaminopyrazolderivate sowie 2,4,5,6-Tetraaminopyrimidin und dessen Derivate eingesetzt. Als Kupplerkomponenten werden in der Regel m-Phenylendiamin-derivate, Naphthole, Pyridinderivate, Resorcin und Resorcinderivate, Pyrazolone und m-Aminophenole verwendet.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Substrat aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so dass dann sehr viel schneller eine vielfach unerwünschte Nuancenverschiebung oder gar ein sichtbarer homogener Farbverlust eintritt.

Insbesondere oxidative Haarfärbemittel sind trotz ihrer vorteilhaften Färbeeigenschaften für den Anwender mit Nachteilen behaftet.

Erstens führt der Einsatz der Oxidationsmittel zur Ausfärbung beziehungsweise Entwicklung der eigentlichen Färbung zu Schädigungen in der Haarstruktur und auf der Haaroberfläche. Das Haar wird brüchig, seine Elastizität lässt nach und die Kämmbarkeit nimmt ab. Zweitens benötigen oxidative Färbemittel in der Regel einen basischen pH-Wert zur Ausfärbung, insbesondere zwischen pH 9,0 und pH 11,5. Diese pH-Werte sind notwendig, um eine Öffnung der äußeren Schuppenschicht (Cuticula) zu gewährleisten und eine Penetration der aktiven Spezies (Farbstoffvorprodukte und/oder Wasserstoffperoxid) in das Haar zu ermöglichen. Das basische Milieu stellt jedoch einen weiteren Grund der Schädigung für das Haar und dessen Struktur dar, der ebenfalls mit gesteigerter Anwendungszeit an Bedeutung gewinnt. Die Spreizung der äußeren Schuppenschicht führt außerdem zu einem unangenehmen Oberflächenempfinden der Haare und damit zu einer verschlechterten Kämmbarkeit im Nass- und Trockenzustand. Dadurch besteht für den Verbraucher eine gesteigerte Notwendigkeit, zusätzliche Nachbehandlungsmittel wie Konditioniermittel einsetzen.

Ein weiteres Problem stellt sich für den Verbraucher in der Erzielung gleichmäßiger Färbungen. Für gleichmäßige Färbungen ist es insbesondere von Bedeutung, den Startpunkt der Färbeprozesse zu kontrollieren. Diese Problematik ist insbesondere dann ausgeprägt, wenn viele ausgewählte, über den Kopf verteilte Haarpartien gefärbt werden sollen. Häufig werden Färbemittel dazu benutzt, nur einzelne Haarsträhnen aufzuhellen, um eine optisch interessante Haarfärbung zu erzeugen. Beim herkömmlichen "Strähnchenprozess" werden einzelnen Haarsträhnen in Folien eingelegt, mit der Färbezubereitung beaufschlagt und in die Folie eingeschlagen. Da der Applikationsprozess langwierig ist, sind die Einwirkzeiten auf die nacheinander beaufschlagten Strähnen unterschiedlich, was zu unerwünschten weil unterschiedlichen Färbeergebnissen mit verschieden gefärbten Strähnen führt. Daher ist eine größere zeitliche Unabhängigkeit für den Anwender, insbesondere für den professionellen Anwender, beim Auftragen und Ausspülen der Färbemittel wünschenswert.

Es hat daher nicht an Versuchen gefehlt, geeignete Methoden zur Verzögerung des Färbebeginns zu entwickeln.

Eine Herangehensweise stellt die selektive Verkapselung von Inhaltsstoffen dar. Die Druckschriften EP1820487A1 und WO2005/044208A1 offenbaren dabei beschichtete, farbstoffhaltige Pellets. Aus der EP1726289A2 sind verkapselte Reduktionsmittel in Färbemitteln bekannt. Die Umhüllung von pH-Stellmitteln in Haarfärbemitteln ist aus der EP1752191A2, die umhüllte Alkalisierungsmittel offenbart, und aus der WO89/06531A1, die verkapselte Acidificierungsmittel beschreibt, bekannt.

Der vorliegenden Erfindung lag die Aufgabe zugrunde, Mittel zum Färben von Keratinfasern bereitzustellen, die es ermöglichen ein gleichmäßiges und einheitliches Färbeergebnis, insbesondere auch bei der "Strähnchentechnik", zu erzielen. Das Überfärben einzelner Strähnen, die im Prozess vor anderen Strähnen mit der Applikationsmischung beaufschlagt werden, sollte vermieden werden.

Es wurde nun gefunden, dass sich die vorstehenden Aufgaben lösen lassen, indem Färbemittel bereitgestellt werden, in denen mindestens ein partikelförmiger Bestandteil enthalten ist, welcher einen Partikelkern aus Oxidationsfarbstoffvorprodukten und eine Beschichtung aus bestimmten Hüllstoffen und Trennmitteln aufweist.

Durch die Beschichtung der Oxidationsfarbstoffvorprodukte wird der Start des eigentlichen Färbevorgangs verzögert, und somit eine größere zeitliche Unabhängigkeit beim Auftragen des Färbemittels für den Anwender erreicht. Auch im Falle einer schlechten Zeiteinhaltung seitens des Anwenders beim Ausspülen der Strähnen wird daher eine gleichmäßige und damit schonende Färbung erhalten.

Als ersten Gegenstand betrifft die vorliegende Erfindung daher ein Mittel zum Färben von keratinischen Fasern, insbesondere menschlichen Haaren, enthaltend mindestens einen partikelförmigen Bestandteil, der einen Partikelkern, enthaltend mindestens ein Oxidationsfarbstoffvorprodukt, umfasst und eine diesen Kern umgebende Hülle aufweist, dadurch gekennzeichnet, dass die Hülle aus mindestens einem Verkapselungsmaterial, ausgewählt unter
a) Homo- und/oder Copolymeren von Methacrylsäure und/oder Methacrylsäureestern (Methacrylaten) und/oder
b) Homo- und/oder Copolymeren von Vinylacetat und/oder
c) Schellack
und mindestens einem Trennmittel, ausgewählt aus der Gruppe, die gebildet wird aus Partialglyceriden von Fettsäuren, Metallseifen und anorganischen, pulverförmigen Trennmitteln, ausgewählt aus Graphit, Talk und Glimmer, besteht.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten nichts entgegen.

Erfindungsgemäße Mittel können in einer Vielzahl von Angebotsformen bereitgestellt werden, beispielsweise in Form von Pasten, Pulvern, Tabletten usw., solange sie mindestens einen partikelförmigen Inhaltsstoff enthalten, welcher mit einer Hülle umgeben ist. Besonders bevorzugt liegen die erfindungsgemäßen Mittel im Hinblick auf die Anwendungskonvenienz jedoch als Färbepulver vor. Erfindungsgemäße Mittel, die als Partikelgemisch ("in Pulverform") vorliegen, sind bevorzugte Ausführungsformen der vorliegenden Erfindung.

Erfindungsgemäße Mittel enthalten mindestens einen partikelförmigen Kern, welcher mindestens ein Oxidationsfarbstoffvorprodukt enthält. Als ersten zwingenden Inhaltsstoff enthalten die erfindungsgemäßen Mittel daher mindestens ein Oxidationsfarbstoffvorprodukt.

Oxidationsfarbstoffvorprodukte können aufgrund ihres Reaktionsverhaltens in zwei Kategorien eingeteilt werden, sogenannte Entwicklerkomponenten und Kupplerkomponenten.

Entwicklerkomponenten können mit sich selbst den eigentlichen Farbstoff ausbilden. Sie können daher als alleinige, farbverändernde Verbindungen im erfindungsgemäßen Mittel enthalten sein. In einer bevorzugten Ausführungsform enthalten die erfindungsgemäßen Mittel mindestens ein Oxidationsfarbstoffvorprodukt vom Entwicklertyp und/oder Kupplertyp. Vorzugsweise enthalten die erfindungsgemäßen Färbemittel mindestens ein Oxidationsfarbstoffvorprodukt vom Entwicklertyp und mindestens ein Oxidationsfarbstoffvorprodukt vom Kupplertyp.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Hydrobromide oder der Sulfate einzusetzen.

Bevorzugte p-Phenylendiamine als Oxidationsfarbstoffvorprodukte vom Entwicklertyp werden ausgewählt aus einer oder mehrerer Verbindungen der Gruppe, die gebildet wird, aus 1,4-Diaminobenzol (p-Phenylendiamin), 1,4-Diamino-2-methylbenzol (p-Toluylendiamin), 1,4-Diamino-2-chlorbenzol (2-Chlor-p-phenylendiamin), 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)anilin, N,N-Bis-(2-hydroxyethyl)-p-phenylendiamin, 4-N,N-Bis-(2-hydroxy-ethyl)amino-2-methylanilin, 4-N,N-Bis-(2-hydroxyethyl)amino-2-chloranilin, 2-(2-Hydroxyethyl)-p-phenylendiamin, 2-(1,2-Dihydroxyethyl)-p-phenylendiamin, 2-Fluor-p-phenylendiamin, 2-Iso-propyl-p-phenylendiamin, N-(2-Hydroxypropyl)-p-phenylendiamin, 2-Hydroxymethyl-p-phenylen-diamin, N,N-Dimethyl-3-methyl-p-phenylendiamin, N-Ethyl-N-2-hydroxyethyl-p-phenylendiamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetylaminoethyloxy)-p-phenylendiamin, N-(2-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen. Erfindungsgemäß besonders bevorzugte p-Phenylendiamin-derivate sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)-propyl]amin, 2-Methoxymethyl-p-phenylendiamin sowie deren physiologisch verträglichen Salzen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten werden aus mindestens einer der folgenden Verbindungen ausgewählt: N,N'-Bis-(2-hydroxyethyl)-N,N'-bis-(4'-aminophenyl)-1,3-diaminopropan-2-ol, N,N'-Bis-(2-hydroxyethyl)-N,N'-bis-(4'-aminophenyl)-ethylendiamin, N,N'-Bis-(4'-aminophenyl)tetramethylendiamin, N,N'-Bis-(2-hydroxyethyl)-N,N'-bis-(4'-aminophenyl)tetramethylendiamin, N,N'-Bis-(4-(methylamino)phenyl)tetramethylendiamin, N,N'-Diethyl-N,N'-bis-(4'-amino-3'-methylphenyl)ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan sowie ihre physiologisch verträglichen Salze. Besonders bevorzugte zweikernige Entwicklerkomponenten werden ausgewählt unter N,N'-Bis-(2-hydroxyethyl)-N,N'-bis-(4-amino-phenyl)-1,3-diaminopropan-2-ol, Bis-(2-hydroxy-5-aminophenyl)methan, 1,3-Bis-(2,5-diamino-phenoxy)propan-2-ol, N,N'-Bis-(4-aminophenyl)-1,4-diazacycloheptan, 1,10-Bis-(2,5-diamino-phenyl)-1,4,7,10-tetraoxadecan oder eines deren physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind 4-Aminophenol, N-Methyl-4-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(2-hydroxyethoxy)phenol, 4-Amino-2-methylphenol, 4-Amino-2-hydroxymethylphenol, 4-Amino-2-methoxymethylphenol, 4-Amino-2-aminomethylphenol, 4-Amino-2-(2-hydroxyethyl-aminomethyl)phenol, 4-Amino-2-(1,2-dihydroxyethyl)phenol, 4-Amino-2-fluorphenol, 4-Amino-2-chlorphenol, 4-Amino-2,6-dichlorphenol, 4-Amino-2-(diethylaminomethyl)phenol sowie ihre physiologisch verträglichen Salze. Besonders bevorzugte Verbindungen sind 4-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise aus Pyrimidinderivaten, Pyrazolderivaten, Pyrazolopyrimidin-Derivaten bzw. ihren physiologisch verträglichen Salzen. Bevorzugte Pyrimidinderivate sind die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin. Bevorzugte Pyrazolderivate sind die Verbindungen, die ausgewählt werden unter 4,5-Diamino-1-methylpyrazol, 4,5-Diamino-1-(2-hydroxyethyl)pyrazol, 3,4-Diamino-pyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydra-zinopyrazol, 1-Benzyl-4,5-diamino-3-methylpyrazol, 4,5-Diamino-3-t-butyl-1-methylpyrazol, 4,5-Diamino-1-t-butyl-3-methylpyrazol, 4,5-Diamino-1-(2-hydroxyethyl)-3-methylpyrazol, 4,5-Diamino-1-ethyl-3-methylpyrazol, 4,5-Diamino-1-ethyl-3-(4-methoxyphenyl)pyrazol, 4,5-Diamino-1-ethyl-3-hydroxymethylpyrazol, 4,5-Diamino-3-hydroxymethyl-1-methylpyrazol, 4,5-Diamino-3-hydroxy-methyl-1-isopropylpyrazol, 4,5-Diamino-3-methyl-1-isopropylpyrazol, 4-Amino-5-(2-aminoethyl)-amino-1,3-dimethylpyrazol, sowie deren physiologisch verträglichen Salze, insbesondere 4,5-Diamino-1-(2-hydroxyethyl)pyrazol. Unter den Pyrazolo[1,5-a]pyrimidinen kann man insbesondere nennen: Pyrazolo[1,5-a]pyrimidin-3,7-diamin, 2,5-Dimethylpyrazolo[1,5-a]pyrimidin-3,7-diamin, Pyrazolo[1,5-a]pyrimidin-3,5-diamin, 2,7-Dimethylpyrazolo[1,5-a]pyrimidin-3,5-diamin, 3-Amino-pyrazolo[1,5-a]pyrimidin-7-ol, 3-Aminopyrazolo[1,5-a]pyrimidin-5-ol, 2-(3-Aminopyrazolo[1,5-a]-pyrimidin-7-ylamino)ethanol, 2-(7-Aminopyrazolo[1,5-a]pyrimidin-3-ylamino)ethanol, 2-[(3-Amino-pyrazolo[1,5-a]pyrimidin-7-yl)-(2-hydroxyethyl)amino]ethanol, 2-[(7-Aminopyrazolo[1,5-a]-pyrimidin-3-yl)-(2-hydroxyethyl)amino]ethanol, 5,6-Dimethylpyrazolo[1,5-a]pyrimidin-3,7-diamin, 2,6-Dimethylpyrazolo[1,5-a]pyrimidin-3,7-diamin, 3-Amino-7-dimethylamino-2,5-dimethylpyrazolo-[1,5-a]pyrimidin sowie ihre physiologisch verträglichen Salze und ihre tautomeren Formen, wenn ein tautomeres Gleichgewicht vorhanden ist.

Bevorzugte Entwicklerkomponenten werden ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylen-diamin, 2-Methoxymethyl-p-phenylendiamin, N-(4-Amino-3-methylphenyl)-N-[3-(1H-imidazol-1-yl)-propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-amino-phenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)-phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, sowie deren physiologisch verträglichen Salzen. Besonders bevorzugte Entwicklerkomponenten sind p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]amin und/oder 4,5-Diamino-1-(2-hydroxy-ethyl)pyrazol sowie deren physiologisch verträglichen Salze.

Die Entwicklerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt.

Bevorzugte 3-Aminophenol-Kupplerkomponenten werden ausgewählt aus mindestens einer Verbindung aus der Gruppe, die gebildet wird aus 3-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-Diethylaminophenol, N-Cyclopentyl-3-aminophenol, 1,3-Dihydroxy-5-(methylamino)benzol, 3-Ethylamino-4-methylphenol, 2,4-Dichlor-3-aminophenol und deren physiologisch verträglichen Salzen. Bevorzugte 3-Diaminobenzol-Kupplerkomponenten werden ausgewählt aus mindestens einer Verbindung aus der Gruppe, die gebildet wird aus 3-Aminoanilin (m-Phenylendiamin), 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)aminobenzol und deren physiologisch verträglichen Salzen. Bevorzugte 1,2-Diaminobenzol-Kupplerkomponenten werden ausgewählt aus mindestens einer Verbindung aus der Gruppe, die gebildet wird aus 3,4-Diaminobenzoesäure und 2,3-Diamino-1-methylbenzol und den physiologisch verträglichen Salzen aller vorstehend genannten Verbindungen. Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol. Bevorzugte Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin und deren physiologisch verträglichen Salzen. Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin. Bevorzugte Indolderivate werden ausgewählt aus mindestens einer Verbindung der Gruppe, die gebildet wird aus 4-Hydroxyindol, 6-Hydroxyindol und 7-Hydroxyindol und deren physiologisch verträglichen Salzen. Bevorzugte Indolinderivate werden ausgewählt aus mindestens einer Verbindung der Gruppe, die gebildet wird aus 4-Hydroxyindolin, 6-Hydroxyindolin und 7-Hydroxyindolin und deren physiologisch verträglichen Salzen. Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und deren physiologisch verträglichen Salzen.

Erfindungsgemäß besonders bevorzugte Kupplerkomponenten werden ausgewählt unter 3-Aminophenol, 5-Amino-2-methylphenol, 3-Amino-2-chlor-6-methylphenol, 2-Hydroxy-4-amino-phenoxyethanol, 5-Amino-4-chlor-2-methylphenol, 5-(2-Hydroxyethyl)amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)-amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Tri-hydroxybenzol, 2-Amino-3-hydroxypyridin, 3-Amino-2-methylamino-6-methoxypyridin, 2,6-Di-hydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxy-indolin, 7-Hydroxyindolin oder Gemischen dieser Verbindungen oder deren physiologisch verträglichen Salzen. Ganz besonders bevorzugt ist Resorcin, 2-Methylresorcin, 5-Amino-2-methylphenol, 3-Aminophenol, 2-(2,4-Diaminophenoxy)ethanol, 1,3-Bis(2,4-diaminophenoxy)propan, 1-Methoxy-2-amino-4-(2'-hydroxyethylamino)benzol, 2-Amino-3-hydroxypyridin und 1-Naphthol sowie eines deren physiologisch verträglichen Salze.

Die Kupplerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Im Rahmen der vorliegenden Erfindung sind folgende Kombinationen aus Oxidationsfarbstoffvorprodukten vom Entwicklertyp und vom Kupplertyp besonders bevorzugt Mit den als Kombination genannten Oxidationsfarbstoffvorprodukten können jedoch auch noch weitere Farbstoffvorprodukte kombiniert werden: p-Toluylendiamin / Resorcin; p-Toluylendiamin / 2-Methylresorcin; p-Toluylendiamin / 5-Amino-2-methylphenol; p-Toluylendiamin / 3-Aminophenol; p-Toluylendiamin / 2-(2,4-Diaminophenoxy)ethanol; p-Toluylendiamin / 1,3-Bis(2,4-diaminophenoxy)propan; p-Toluylendiamin / 1-Methoxy-2-amino-4-(2-hydroxyethylamino)benzol; p-Toluylendiamin / 2-Amino-3-hydroxypyridin; p-Toluylendiamin / 1-Naphthol; 2-(2-Hydroxyethyl)-p-phenylendiamin / Resorcin; 2-(2-Hydroxyethyl)-p-phenylendiamin / 2-Methylresorcin; 2-(2-Hydroxyethyl)-p-phenylendiamin / 5-Amino-2-methylphenol; 2-(2-Hydroxyethyl)-p-phenylendiamin / 3-Aminophenol; 2-(2-Hydroxy-ethyl)-p-phenylendiamin / 2-(2,4-Diaminophenoxy)ethanol; 2-(2-Hydroxyethyl)-p-phenylendiamin / 1,3-Bis(2,4-diaminophenoxy)propan; 2-(2-Hydroxyethyl)-p-phenylendiamin / 1-Methoxy-2-amino-4-(2-hydroxyethylamino)benzol; 2-(2-Hydroxyethyl)-p-phenylendiamin / 2-Amino-3-hydroxypyridin; 2-(2-Hydroxyethyl)-p-phenylendiamin / 1-Naphthol; 2-Methoxymethyl-p-phenylendiamin / Resorcin; 2-Methoxymethyl-p-phenylendiamin / 2-Methylresorcin; 2-Methoxymethyl-p-phenylendiamin / 5-Amino-2-methylphenol; 2-Methoxymethyl-p-phenylendiamin / 3-Aminophenol; 2-Methoxymethyl-p-phenylendiamin / 2-(2,4-Diaminophenoxy)ethanol; 2-Methoxymethyl-p-phenylendiamin / 1,3-Bis(2,4-diaminophenoxy)propan; 2-Methoxymethyl-p-phenylendiamin / 1-Methoxy-2-amino-4-(2-hydroxyethylamino)benzol; 2-Methoxymethyl-p-phenylendiamin / 2-Amino-3-hydroxypyridin; 2-Methoxymethyl-p-phenylendiamin / 1-Naphthol; N-(4-Amino-3-methylphenyl)-N-[3-(1H-imidazol-1-yl)propyl]amin / Resorcin; N-(4-Amino-3-methylphenyl)-N-[3-(1H-imidazol-1-yl)propyl]amin / 2-Methylresorcin; N-(4-Amino-3-methylphenyl)-N-[3-(1H-imidazol-1-yl)propyl]amin / 5-Amino-2-methylphenol; N-(4-Amino-3-methylphenyl)-N-[3-(1H-imidazol-1-yl)propyl]amin / 3-Aminophenol; N-(4-Amino-3-methylphenyl)-N-[3-(1H-imidazol-1-yl)propyl]amin / 2-(2,4-Diamino-phenoxy)ethanol; N-(4-Amino-3-methylphenyl)-N-[3-(1 H-imidazol-1-yl)propyl]amin / 1,3-Bis(2,4-di-aminophenoxy)propan; N-(4-Amino-3-methylphenyl)-N-[3-(1H-imidazol-1-yl)propyl]amin / 1-Methoxy-2-amino-4-(2-hydroxyethylamino)benzol; N-(4-Amino-3-methylphenyl)-N-[3-(1H-imid-azol-1-yl)propyl]amin / 2-Amino-3-hydroxypyridin; N-(4-Amino-3-methylphenyl)-N-[3-(1H-imidazol-1-yl)propyl]amin / 1-Naphthol; 4,5-Diamino-1-(2-hydroxyethyl)pyrazol / Resorcin; 4,5-Diamino-1-(2-hydroxyethyl)pyrazol / 2-Methylresorcin; 4,5-Diamino-1-(2-hydroxyethyl)pyrazol / 5-Amino-2-methylphenol; 4,5-Diamino-1-(2-hydroxyethyl)pyrazol / 3-Aminophenol; 4,5-Diamino-1-(2-hydroxy-ethyl)pyrazol / 2-(2,4-Diaminophenoxy)ethanol; 4,5-Diamino-1-(2-hydroxyethyl)pyrazol / 1,3-Bis-(2,4-diaminophenoxy)propan; 4,5-Diamino-1-(2-hydroxyethyl)pyrazol / 1-Methoxy-2-amino-4-(2-hydroxyethylamino)benzol; 4,5-Diamino-1-(2-hydroxyethyl)pyrazol / 2-Amino-3-hydroxypyridin; 4,5-Diamino-1-(2-hydroxyethyl)pyrazol / 1-Naphthol.

Um eine ausgewogene und subtile Nuancenausbildung zu erzielen, ist es erfindungsgemäß vorteilhaft, wenn weitere farbgebende Komponenten im erfindungsgemäßen Mittel enthalten sind.

In einer weiteren Ausführungsform können die erfindungsgemäßen Mittel zusätzlich mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Es kann erfinderisch bevorzugt sein, wenn der Partikelkern neben den Oxidationsfarbstoffvorprodukten weitere Komponenten umfasst, wie beispielsweise Streckmittel und/oder Trägerstoffe.

Als weiteres Merkmal der vorliegenden Erfindung ist der Oxidationsfarbstoffvorprodukte enthaltende Partikelkern von einer Hülle umgeben, die aus mindestens einem Verkapselungsmaterial, ausgewählt unter
a) Homo- und/oder Copolymeren von Methacrylsäure und/oder Methacrylsäureestern (Methacrylaten) und/oder
b) Homo- und/oder Copolymeren von Vinylacetat und/oder
c) Schellack
und mindestens einem Trennmittel, ausgewählt aus der Gruppe, die gebildet wird aus Partialglyceriden von Fettsäuren, Metallseifen und anorganischen, pulverförmigen Trennmitteln, ausgewählt aus Graphit, Talk und Glimmer, besteht.

In erfindungsgemäß bevorzugten Mitteln besteht die Hülle vorzugsweise zu mindestens 50 Gew.-%, weiter bevorzugt zu mindestens 70 Gew.-% und insbesondere zu mindestens 80 Gew.-% ihres Gewichts aus
a) Homo- und/oder Copolymeren von Methacrylsäure und/oder Methacrylsäureestern und/oder
b) Homo- und/oder Copolymeren von Vinylacetat und/oder
c) Schellack.

Erfindungsgemäß besonders geeignete Verkapselungsmittel sind die Homo- und/oder Copolymere von Methacrylsäure und/oder Methacrylsäureestern. Unter diesen sind die Copolymere von Methacrylsäure und/oder Methacrylsäureestern bevorzugt. Besonders bevorzugte Vertreter werden nachstehend beschrieben.

Ein besonders geeignetes Hüllmaterial stellen Copolymere aus Methacrylsäure, Methylacrylat und Methylmethacrylat dar. Diese enthalten Monomereinheiten der folgenden Strukturen: 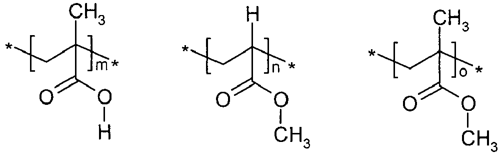 wobei die Indices m, n, und o je nach Molmasse des Polymers variieren und nicht bedeuten, dass es sich um Blockcopolymere handelt. Vielmehr können Struktureinheiten im Molekül statistisch verteilt vorliegen.

In erfindungsgemäß bevorzugten Mitteln enthält die Hülle mindestens ein Copolymer aus Methacrylsäure, Methylacrylat und Methylmethacrylat.

In besonders bevorzugten Polymeren dieses Typs ist das molare Verhältnis von Methacrylsäure zu den beiden Estern > 1, vorzugsweise > 5. Besonders bevorzugte erfindungsgemäße Mittel sind dadurch gekennzeichnet, dass das molare Verhältnis von Estergruppen zu freien Carboxygruppen im Copolymer 1:5 bis 1:12 beträgt. Noch weiter bevorzugte Polymere dieses Typs weisen Molmassen um 220 kDa auf. Besonders bevorzugte erfindungsgemäße Mittel sind dadurch gekennzeichnet, dass das Copolymer aus Methacrylsäure, Methylacrylat und Methylmethacrylat eine Molmasse von 200 bis 250 kDa aufweist.

Ein weiteres besonders geeignetes Hüllmaterial stellen Copolymere aus Methacrylsäure, und Ethylacrylat dar. Diese enthalten Monomereinheiten der folgenden Strukturen: 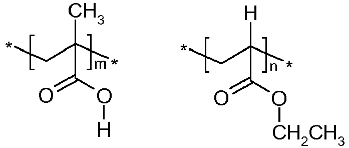 wobei die Indices m und n je nach Molmasse des Polymers variieren und nicht bedeuten, dass es sich um Blockcopolymere handelt. Vielmehr können Struktureinheiten im Molekül statistisch verteilt vorliegen.

In erfindungsgemäß bevorzugten Mitteln enthält die Hülle mindestens ein Copolymer aus Methacrylsäure und Ethylacrylat. In besonders bevorzugten Polymeren dieses Typs ist das molare Verhältnis von Methacrylsäure zu dem Ester ca. 1. Besonders bevorzugte erfindungsgemäße Mittel sind dadurch gekennzeichnet, dass das molare Verhältnis von Estergruppen zu freien Carboxygruppen im Copolymer 1:0,9 bis 0,9:1 beträgt.

Noch weiter bevorzugte Polymere dieses Typs weisen Molmassen um 250 kDa auf. Besonders bevorzugte erfindungsgemäße Mittel sind dadurch gekennzeichnet, dass das Copolymer aus Methacrylsäure und Ethylacrylat eine Molmasse von 225 bis 275 kDa aufweist.

Ein weiteres besonders geeignetes Hüllmaterial stellen Copolymere dar, welche Methacrylsäureester mit kationischen Gruppen enthalten, insbesondere aus (ω-Trialkylammonioalkyl)methacrylate. Besonders bevorzugt sind erfindungsgemäße Mittel, bei denen die Hülle mindestens ein Copolymer aus (2-Trimethylammonioethyl)methacrylat chlorid enthält. Dieses enthält Monomereinheiten der Formel (I)

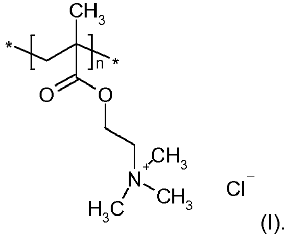

Als zusätzliche(s) Monomer(e) in Copolymeren mit Monomereinheiten der Formel (I) haben sich insbesondere Ester der Methacrylsäure und/oder Ester der Acrylsäure bewährt.

In bevorzugten Mittel enthält die Hülle der Partikel daher Copolymere aus (2-Trimethylammonio-ethyl)methacrylat chlorid und mindestens einem zusätzlichen Monomer, welches ausgewählt wird aus der Gruppe, die gebildet wird aus Ethylmethacrylat, Methylmethacrylat, Ethylacrylat und Methylacrylat.

Bevorzugt sind erfindungsgemäße Mittel, bei denen das Copolymer mit Monomereinheiten der Formel (I) als zusätzliche(s) Monomer(e) Ethylmethacrylat enthält.

Weiter bevorzugt sind erfindungsgemäße Mittel, bei denen das Copolymer mit Monomereinheiten der Formel (I) als zusätzliche(s) Monomer(e) Methylmethacrylat enthält.

Zusätzlich zu den weiteren Methacrylsäureestern oder an deren Stelle können auch Acrylsäureester in das Copolymer mit Monomereinheiten der Formel (I) einpolymerisiert werden. Hier sind erfindungsgemäße Mittel bevorzugt, bei denen das Copolymer mit Monomereinheiten der Formel (I) als zusätzliche(s) Monomer(e) Ethylacrylat enthält.

Weiter bevorzugt sind erfindungsgemäße Mittel, bei denen das Copolymer mit Monomereinheiten der Formel (I) als zusätzliche(s) Monomer(e) Methylacrylat enthält.

Ein weiteres besonders geeignetes Hüllmaterial stellen Copolymere aus Methylmethacrylat und Ethylacrylat dar. Diese enthalten Monomereinheiten der folgenden Strukturen: 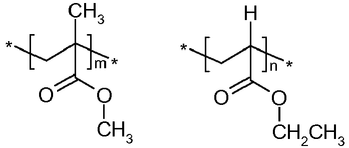 wobei die Indices m und n je nach Molmasse des Polymers variieren und nicht bedeuten, dass es sich um Blockcopolymere handelt. Vielmehr können Struktureinheiten im Molekül statistisch verteilt vorliegen.

In erfindungsgemäß bevorzugten Mitteln enthält die Hülle mindestens ein Copolymer aus Methylmethacrylat und Ethylacrylat.

In besonders bevorzugten Polymeren dieses Typs ist das molare Verhältnis von Methacrylsäure zu dem Ester ca. 1. Besonders bevorzugte erfindungsgemäße Mittel sind dadurch gekennzeichnet, dass das molare Verhältnis von Estergruppen zu freien Carboxygruppen im Copolymer 1:0,9 bis 0,9:1 beträgt.

Noch weiter bevorzugte Polymere dieses Typs weisen Molmassen um 800 kDa auf. Besonders bevorzugte erfindungsgemäße Mittel sind dadurch gekennzeichnet, dass das Copolymer aus Methylmethacrylat und Ethylacrylat eine Molmasse von 750 bis 850 kDa aufweist.

Bevorzugte Acrylsäure-Methacrylsäure-Methacrylsäureester-Copolymertypen werden von der Firma BASF unter den Handelsnamen Eudragit FS 30D, Eudragit L30D-55 (= Kollicoat MAE 30DP), Eudragit RS 30D, Eudragit RL 30D und Eudragit NE 30D vertrieben.

Erfindungsgemäß besonders geeignete Verkapselungsmittel sind weiterhin Homo- und/oder Copolymere von Vinylacetat. Unter den Copolymeren von Vinylacetat sind insbesondere dessen Copolymere mit Ethylen (Ethylen-Vinylacetat-Copolymere, Kurzzeichen EVA oder EVAC) und/oder mit Vinylchlorid [Kurzzeichen VCEVAC (oft auch fälschlich VCEVA) bzw. VCVAC (auch PVCA)] von Bedeutung und können erfindungsgemäß eingesetzt werden.

Besonders bevorzugt sind jedoch erfindungsgemäße Mittel, die das Hompolymer als Beschichtungsmaterial für die Partikel beinhalten, bei denen die Hülle Polyvinylacetat enthält.

Polyvinylacetat (Kurzzeichen PVAC) wird durch radikalische Polymerisation von Essigsäurevinylester (Vinylacetat) erhalten. Die Verknüpfung der Monomeren beim Aufbau der Polymerkette erfolgt in hohen Anteilen (bis zu 98%) als Kopf/Schwanz-Polymerisation und nur in geringem Maß als Kopf/Kopf-Polymerisation; die Makromoleküle der Polyvinylacetate enthalten also hauptsächlich Gruppierungen des Typs I (Kopf/Schwanz) und nur wenige des Typs II (Kopf/Kopf) als charakteristische Grundeinheiten:

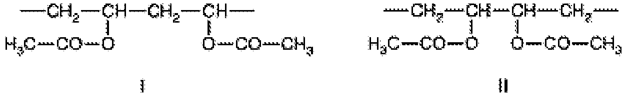

Die Herstellung der Polyvinylacetate kann nach Verfahren der Substanz-, Lösungs-, Sus-ρensions- (Perl-) oder als Emulsionspolymerisation erfolgen. Bevorzugte Polyvinylacetate haben Molmassen von 10.000 - 1.500.000 g/mol und eine Molmassen-abhängige Glasübergangs-temperatur von ca. 28°C. Sie sind amorphe, geruch- und geschmacklose Produkte mit hoher Licht- und Witterungs-Beständigkeit, unlöslich in Wasser und löslich in vielen organischen Lösungsmitteln (Ester, Ether, Ketone, halogenierte Kohlenwasserstoffe u.a.). Ein besonders geeignetes Polyvinylacetat wird unter dem Handelsnamen Kollicoat SR30D von der Firma BASF vertrieben.

Ein erfindungsgemäß besonders geeignetes Verkapselungsmittel ist weiterhin Schellack. Schellack ist aus dem Sekret von Lackschildläusen (*Kerria lacca* bzw. *Laccifer lacca*) gewinnbar und fällt als zähes Harz mit einer durchschnittlichen Molmasse von ca. 1000 g/mol an. Es besteht überwiegend aus teilweise ungesättigten Hydroxycarbonsäuren, die miteinander verestert oder als Lacton vorliegen. Hauptkomponenten sind dabei Aleuritinsäure (Aleurinsäure, 9,10,16-Trihydroxypalmitinsäure) mit bis zu ca. 32 Gew.-% und Shellolsäure.

Schellack ist gut löslich in Alkoholen, organischen Säuren und wässrigen Laugen, weniger in Estern und Ketonen und unlöslich in Kohlenwasserstoffen und Wasser.

Bei der Gewinnung von Schellack wird auf Zweigen abgesondertes Sekret gesammelt, von Zweigresten befreit und alkalisch entfärbt. Aus diesem sogenannten Körnerlack wird der eigentliche Schellack als wachshaltiges oder wachsfreies Harz isoliert.

Der nach einem Schmelzfiltrationsverfahren, bei dem aufgeschmolzener Körnerlack zur Abtrennung von Begleitstoffen filtriert wird, hergestellte Schellack (Handelsnamen: Lemon, TN, Ivory, Orange, Honey) hat noch den natürlichen Wachsanteil von ca. 4 bis 6 Gew.-%.

Gebleichter Schellack fällt bei der Einwirkung von Chlorbleichlauge (Natriumhypochlorit) auf Körnerlack als weißes Pulver an; er wird wachshaltig oder wachsfrei angeboten.

Aus Körnerlack durch Lösungsmittel-Extraktion unter (partieller) Entfärbung mit Aktivkohle gewonnener wachsfreier Schellack, der beim Trocknen in Form dünner Blättchen anfällt, wird als Blätterschellack gehandelt. Solch wachsfreier Schellack wird beispielsweise unter dem Handelsnamen Shellac SSB 55 Astra FL (Wachsgehalt maximal 0,2 Gew.-%), Shellac CZSH 2 (Wachsgehalt maximal 0,25 Gew.-%), Mantrolac R 49 (Wachsgehalt maximal 0,2 Gew.-%) und Angelo Dewaxed Garnet Shellac vertrieben.

Erfindungsgemäß besonders bevorzugt wird wachsfreier bzw. wachsarmer Schellack als Verkapselungsmaterial verwendet. Unter wachsarmen Schellack wird hierbei Schellack verstanden, dessen Gewichtsanteil an Wachs maximal 0,5 Gew.-% beträgt.

Über die große Anzahl seiner funktionellen Gruppen ist Schellack leicht härtbar und chemisch modifizierbar. Er lässt sich insbesondere mit weiteren polymeren Verkapselungsmittel gut mischen.

Die Verkapselungsmittel sind miteinander mischbar und können zur Erreichung einer bestimmten Zeitverzögerung miteinander kombiniert werden.

Weiterhin enthält die den Partikelkern umgebende Hülle neben dem Verkapselungsmaterial mindestens ein Trennmittel. Das Trennmittel dient dazu, ein Verkleben oder Verbacken der umhüllten Partikel im Herstellprozess und/oder während der Lagerung und/oder gegebenenfalls während dem Vermischen mit weiteren Zubereitungen zu verhindern.
Erfindungsgemäß bevorzugte Trennmittel werden ausgewählt aus der Gruppe, die gebildet wird aus Partialglyceriden von Fettsäuren, Metallseifen und anorganischen, pulverförmigen Trennmitteln, ausgewählt aus Graphit, Talk und Glimmer.
Als erfindungsgemäße Trennmittel geeignete Partialglyceride von Fettsäuren sind insbesondere Mono- und Difettsäureglycerylester. Beispiele solcher Partialglyceride sind Glycerinmonostearat, Glycerindistearat, Glycerinmonooleat, Glycerindioleat, Glycerinmonodecanoat, Glycerindidecanoat, Glycerinmonolaurat, Glycerindilaurat, Glycerinmonomyristat, Glycerindimyristat, Glycerinmonopalmitat oder Glycerindipalmitat. Erfindungsgemäß geeignete Partialglyceride von Fettsäuren leiten sich auch von natürlich oder synthetisch anfallenden Fettsäuregemischen ab. Beispiele hierfür sind insbesondere Glycerinmonococoat, Glycerindicocoat, Glycerinmonocetearat, Glycerindicetearat, Glycerinmonotalgester und Glycerinditalgester. Ein besonders bevorzugtes Trennmittel ist Glycerinmonostearat.

Als erfindungsgemäße Trennmittel geeignete Metallseifen sind insbesondere die Seifen mehrwertiger Kationen, insbesondere von Calcium-, Blei-, Magnesium-, Aluminium- und Zink-Kationen. Als Fettsäuren kommen insbesondere Laurinsäure, Myristinsäure, Palmitinsäure, Ölsäure und Stearinsäure in Frage. Besonders bevorzugt sind Seifen, die sich von Stearinsäure ableiten. Erfindungsgemäß besonders bevorzugte Metallseifen sind Magnesiumstearat, Calciumstearat und Zinkstearat, wobei Magnesiumstearat besonders bevorzugt ist.

Weiterhin erfindungsgemäße Trennmittel sind anorganische, pulverförmige Trennmittel, ausgewählt aus Graphit, Talk und Glimmer. Ein bevorzugtes anorganisches, pulverförmiges Trennmittel ist Talk (Magnesiumsilikat).

Erfindungsgemäße besonders bevorzugte Mittel sind daher dadurch gekennzeichnet, die Hülle als Trennmittel Talk enthält.

In erfindungsgemäß bevorzugten Mitteln besteht die Hülle vorzugsweise zu höchstens 50 Gew.-%, weiter bevorzugt zu höchstens 30 Gew.-% und insbesondere zu 20 Gew.-% ihres Gewichts aus einem oder mehreren Trennmitteln.

Sofern die Hülle nicht ausschließlich aus Verkapselungsmitteln und Trennmitteln besteht, kann sie weitere Inhaltsstoffe wie Farb- und Duftstoffe oder Hilfsstoffe enthalten.

Besonders bevorzugte erfindungsgemäße Mittel enthalten in der Hülle der Partikel Weichmacher für eine bessere Elastizität der Hülle. Weichmacher stammen bevorzugt aus der Gruppe Dialkylphthalat, insbesondere Diethylphthalat, Triethylcitrat, Glycerintriacetat und/oder der Polyethylenglycole.

In einer weiteren Ausführungsform enthalten die erfindungsgemäßen Mittel beschichtete Partikel, deren den Partikelkern umgebende Hülle zusätzlich mindestens ein Desintegrationsmittel enthält.

Derartige Desintegrationsmittel werden in der Literatur häufig auch als Zerfallsmittel oder Sprengmittel beschrieben. Derartige Substanzen werden in die Polymerumhüllung eingearbeitet, um deren Zerfallszeiten zu verkürzen. Dieser Zerfall oder diese Sprengung geschieht insbesondere durch eine Volumenvergrößerung infolge von Wasserzutritt (Quellung).

Unter solchen Desintegrationsmitteln bzw. Zerfallsbeschleunigern werden Hilfsstoffe verstanden, die für den raschen Zerfall von Formkörpern in Wasser sorgen.

Als bevorzugte Desintegrationsmittel werden im Rahmen der vorliegenden Erfindung Desintegrationsmittel auf Cellulosebasis eingesetzt. Geeignete Cellulosen bestehen dabei aus ca. 500 bis 5000 Glucose-Einheiten und haben demzufolge durchschnittliche Molmassen von 50.000 bis 500.000. Als Desintegrationsmittel auf Cellulosebasis verwendbar sind im Rahmen der vorliegenden Erfindung auch Cellulose-Derivate, die durch polymeranaloge Reaktionen aus Cellulose erhältlich sind. Solche chemisch modifizierten Cellulosen umfassen dabei beispielsweise Produkte aus Veresterungen bzw. Veretherungen, in denen Hydroxy-Wasserstoffatome substituiert wurden. Aber auch Cellulosen, in denen die Hydroxy-Gruppen gegen funktionelle Gruppen, die nicht über ein Sauerstoffatom gebunden sind, ersetzt wurden, lassen sich als Cellulose-Derivate einsetzen. In die Gruppe der Cellulose-Derivate fallen beispielsweise Alkalicellulosen, Carboxymethylcellulose (CMC), Celluloseester und -ether sowie Aminocellulosen. Die genannten Cellulosederivate werden vorzugsweise nicht als einzige Desintegrationsmittel auf Cellulosebasis eingesetzt, sondern in Mischung mit Cellulose verwendet. Der Gehalt dieser Mischungen an Cellulosederivaten beträgt vorzugsweise unterhalb 50 Gew.-%, besonders bevorzugt unterhalb 20 Gew.-%, bezogen auf das Desintegrationsmittel auf Cellulosebasis. Besonders bevorzugt wird als Desintegrationsmittel auf Cellulosebasis reine Cellulose eingesetzt, die frei von Cellulosederivaten ist. Geeignete Carboxymethylcellulose-Derivate werden beispielsweise unter dem Handelsnamen Tylopur von der Firma Clariant oder Ac-Di-Sol der Firma FMC vertrieben.

Als weiteres Desintegrationsmittel auf Cellulosebasis oder als Bestandteil dieser Komponente kann mikrokristalline Cellulose verwendet werden. Diese mikrokristalline Cellulose wird durch partielle Hydrolyse von Cellulosen unter solchen Bedingungen erhalten, die nur die amorphen Bereiche (ca. 30% der Gesamt-Cellulosemasse) der Cellulosen angreifen und vollständig auflösen, die kristallinen Bereiche (ca. 70%) aber unbeschadet lassen. Eine nachfolgende Desaggregation der durch die Hydrolyse entstehenden mikrofeinen Cellulosen liefert die mikrokristallinen Cellulosen, die Primärteilchengrößen von ca. 5 µm aufweisen und beispielsweise zu Granulaten mit einer mittleren Teilchengröße von 200 µm kompaktierbar sind. Geeignete mikrokristalline Cellulose ist beispielsweise unter den Handelsnamen Emcocel von der Firma JRS Pharma oder Avicel von der Firma FMC kommerziell erhältlich.

Weiterhin kann bevorzugt auch Stärke als Desintegrationsmittel im Rahmen der vorliegenden Erfindung eingesetzt werden. Die erfindungsgemäß einsetzbare Stärke wird üblicherweise aus pflanzlichen Rohstoffen, wie Reis, Soja, Kartoffeln oder Mais gewonnen. Stärke kann unmodifiziert oder analog zur Cellulose als modifizierte Stärke eingesetzt werden. Besonders bevorzugte Stärke-Modifikationen liefern dabei Veresterungs- und Veretherungsreaktionen, insbesondere die erhaltenen Ether aus Reaktionen mit Hydroxycarbonsäuren. Eine erfindungsgemäß besonders geeignete Stärkemodifikation ist die Mischung aus Natriumcarboxymethylstärke und Natriumglykolstärke, die unter dem Handelsnamen Explotab durch die Firma JRS Pharma vertrieben wird.

Erfindungsgemäß besonders bevorzugt sind Desintegrationsmittel auf Maisstärke-Basis. Geeignete, modifizierte Maisstärken sind beispielsweise unter den Handelsnamen Glycolys von der Firma Roquette oder Starch 1500 von der Firma Colorcon erhältlich.

Schließlich stellen Desintegrationsmittel aus vernetztem, wasserunlöslichen Polyvinylpyrrolidinon (PVP) eine weitere Klasse erfindungsgemäß besonders geeigneter Desintegrationsmittel dar. Die vorteilhafte Vernetzung dieser PVP-Modifikation beruht dabei vornehmlich auf Verwicklungen und Verschlingungen der einzelnen Polymerstränge ineinander. Ein erfindungsgemäß besonders bevorzugtes Desintegrationsmittel auf PVP-Basis wird unter dem Handelsnamen Kollidon CL durch die Firma BASF vertrieben.

Die erfindungsgemäßen, den Partikelkern umgebenden Hüllen enthalten die Zerfallshilfsmittel insbesondere in Mengen von 0,05 bis 20 Gew.-%, bevorzugt von 0,1 bis 10 Gew.-%, jeweils bezogen auch das Gesamtgewicht der getrockneten Hülle.

In einer weiteren Ausführungsform enthalten die erfindungsgemäßen Mittel beschichtete Partikel, deren den Partikelkern umgebende Hülle zusätzlich mindestens einen Porenbildner enthält.

Porenbildner werden in die Beschichtung mit eingearbeitet und bewirken, dass sich in der Oberfläche der Beschichtung Poren bilden: Dadurch kommt es zu einer Steigerung der Diffusionsrate in die Polymerhülle für hydrophile Substanzen, insbesondere Wasser.

Erfindungsgemäß eignen sich als Porenbildner besonders Polyvinylpyrrolidinon, Zucker und Zuckeralkohole, wie Lactose, Saccharose, Sorbit und Mannit, Polyethylenglykole mit weniger als 600 Ethylenoxideinheiten, sowie Cellulosederivate, wie Hydroxypropylcellulose, Hydroxypropylmethylcellulose, Methylcellulose und deren Mischungen. Erfindungsgemäß besonders bevorzugte Porenbildner sind Polyvinylpyrrolidinone (PVP), die beispielsweise unter dem Handelsnamen Kollidon von der Firma BASF vertrieben werden.

Erfindungsgemäß ist der Gewichtsanteil der Porenbildner in getrockneten Hülle zwischen 0,05 und 20 Gew.-%, insbesondere zwischen 0,1 und 10 Gew.-%, jeweils bezogen auf das Gesamtgewicht der Hülle.

In einer erfindungsgemäßen Herstellung der Hülle werden die Verkapselungsmittel und die Trennmittel sowie gegebenenfalls die Hilfsstoffe homogen vermischt und mittels Wirbelschichttechnologie (mit Bottom Spray Methode mit oder ohne Wurstereinsatz oder Tangential Spray Methode) als wässrige Dispersion auf die Pulverpartikel aus mindestens Oxidationsfarbstoffvorprodukten aufgesprüht und im Luftstrom gleichzeitig abgetrocknet, so dass eine gleichmäßige, nahezu 100%ige Schicht die Pulverpartikel umschließt.

Alternativ zur Wirbelschichttechnologie sind alternativ Beschichtungen mit den oben genannten Filmmaterialien mit Hilfe der Strahlschicht-Technologie möglich. Eine weitere Möglichkeit, Pulverpartikel zu umhüllen, stellt die Schmelzextrusionstechnologie dar.

Es kann erfindungsgemäß vorteilhaft sein, die Partikelkerne mit einer mehrschichtigen Hülle zu verkapseln. Dabei können gleiche oder hinsichtlich ihrer Zusammensetzung an Verkapselungsmaterialien, Trennmitteln und Hilfsstoffen verschiedene Dispersionen nacheinander aufgebracht werden, gegebenenfalls auch mittels unterschiedlicher Beschichtungstechniken aufgetragen werden.

In einer besonderen Ausführungsform der vorliegenden Erfindung hat es sich als besonders vorteilhaft erwiesen, wenn die partikelförmigen Bestandteile als zusätzliches Trennmittel zur besonders effektiven Vermeidung von Verkleben oder Verbacken der Teilchen Siliciumdioxid enthalten. Als zusätzliches Trennmittel kann dabei insbesondere amorphes und/oder pyrogenes Siliciumdioxid eingesetzt werden, welches beispielsweise unter den Handelsnamen Syloid 244FP von der Firma Grace GmbH oder Aerosil von der Firma Evonik vertrieben wird. Dazu können die partikelförmigen Bestandteile direkt mit Siliciumdioxid gemischt werden, insbesondere solange die Partikel noch nicht vollständig getrocknet sind.

Erfindungsgemäß besonders bevorzugt werden die partikelförmigen Bestandteile enthaltend als zusätzliches Trennmittel Siliciumdioxid dadurch hergestellt, dass die bereits mit einer oder mehreren Hüllen beschichteten, partikelförmigen Bestandteile mit einer wässrigen Dispersion, enthaltend Siliciumdioxid, besprüht werden und anschließend getrocknet werden. Eine solche Siliciumdioxid-haltige Dispersion enthält bevorzugt zwischen 5 und 30 Gew.-% Siliciumdioxid und wird beispielsweise unter dem Handelsnamen Aerodisp W von der Firma Evonik vertrieben.

In einer weiteren Ausführungsform werden Teilmengen der Partikelkerne mit unterschiedlichen Hüll- oder Coatingmaterialien verkapselt. Diese Teilchargen werden in einem weiteren Herstellprozessschritt in einem bestimmten Anteil miteinander gemischt.

Die erfindungsgemäßen Mittel enthalten beschichtete Partikel, die einen mittleren Teilchendurchmesser von 50 bis 500 µm besitzen, bevorzugt von 100 µm bis 250 µm.

In weiter bevorzugten Mitteln wird das Beschichtungsmaterial in einer bestimmten Menge auf die zu beschichtenden Partikel aufgebracht. Hier sind erfindungsgemäße Mittel bevorzugt, bei denen die Hülle 10 bis 70 Gew.-% des Gewichts der beschichteten Partikel ausmacht.

Im Falle der oxidativen Färbungen kann die Entwicklung der Farbe grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Dieser Aufhelleffekt kann unabhängig von der Färbemethode gewünscht sein. Als Oxidationsmittel kommen Persulfate, Peroxodisulfate, Chlorite, Hypochlorite und insbesondere Wasserstoffperoxid oder und/oder eines seiner festen Anlagerungsprodukte an organische oder anorganische Verbindungen in Frage.

Um eine vorzeitige, unerwünschte Reaktion der Oxidationsfarbstoffvorprodukte durch das Oxidationsmittel zu verhindern, werden Oxidationsfarbstoffvorprodukte und Oxidationsmittel selbst zweckmäßigerweise getrennt voneinander konfektioniert und erst unmittelbar vor der Anwendung in Kontakt gebracht.

In einer weiteren Ausführungsform der vorliegenden Erfindung sind daher Mittel bevorzugt, welche dadurch gekennzeichnet sind, dass sie unmittelbar vor der Anwendung durch Vermischen mindestens zweier Zubereitungen hergestellt wird, wobei die mindestens zwei Zubereitungen in mindestens zwei getrennt konfektionierten Containern bereitgestellt werden und wobei ein Container (I) eine Färbezubereitung (A), und ein weiterer Container (II) eine Oxidationsmittelzubereitung (B), enthaltend mindestens ein Oxidationsmittel, enthält,
wobei die Färbezubereitung (A) mindestens einen partikelförmigen Bestandteil enthält, der einen Partikelkern, enthaltend mindestens ein Oxidationsfarbstoffvorprodukt, umfasst und eine diesen Kern umgebende Hülle aufweist, dadurch gekennzeichnet, dass die Hülle aus mindestens einem Verkapselungsmaterial, ausgewählt unter
a) Homo- und/oder Copolymeren von Methacrylsäure und/oder Methacrylsäureestern (Methacrylaten) und/oder
b) Homo- und/oder Copolymeren von Vinylacetat und/oder
c) Schellack
und mindestens einem Trennmittel besteht.

Mittel dieser Ausführungsform werden bevorzugt als Mehrkomponenten-Verpackungseinheiten (Kit of Parts) vertrieben.

In einer bevorzugten Ausführungsform liegt die Färbezubereitung (A) in Form eines Pulvers oder feinen Granulats vor. In dieser Ausführungsform wird der Container (I) bevorzugt in Form eines Tütchens, eines Sachets, eines Beutels oder auch in Form einer gegebenenfalls wiederverschließbaren Dose bereitgestellt.

Für den Fall, dass die Färbezubereitung (A) in Form eines Pulvers oder feinen Granulats vorliegt, kann es zur Herstellung des anwendungsbereiten Färbemittels erfindungsgemäß vorteilhaft sein, die Färbezubereitung (A) zunächst mit einer zusätzlichen, flüssigen Creme- oder Emulsionsgrundlage (C) zu vermischen und anschließend eine Oxidationsmittelzubereitung (B) zuzugeben.

In einer weiteren Ausführungsform der vorliegenden Erfindung sind daher Mittel bevorzugt, welche dadurch gekennzeichnet sind, dass sie unmittelbar vor der Anwendung durch Vermischen mindestens dreier Zubereitungen hergestellt wird, wobei die mindestens drei Zubereitungen in mindestens drei getrennt konfektionierten Containern bereitgestellt werden und wobei ein Container (I) eine Färbezubereitung (A), ein Container (la) eine Emulsionszubereitung (C) und ein weiterer Container (II) eine Oxidationsmittelzubereitung (B), enthaltend mindestens ein Oxidationsmittel, enthält,
wobei die Färbezubereitung (A) mindestens einen partikelförmigen Bestandteil enthält, der einen Partikelkern, enthaltend mindestens ein Oxidationsfarbstoffvorprodukt, umfasst und eine diesen Kern umgebende Hülle aufweist, dadurch gekennzeichnet, dass die Hülle aus mindestens einem Verkapselungsmaterial, ausgewählt unter
a) Homo- und/oder Copolymeren von Methacrylsäure und/oder Methacrylsäureestern (Methacrylaten) und/oder
b) Homo- und/oder Copolymeren von Vinylacetat und/oder
c) Schellack
und mindestens einem Trennmittel
besteht, und wobei zunächst die Zubereitungen (A) und (C) innig vermischt werden und anschließend die Zubereitung (B) zugegeben wird.

Bevorzugt enthält die Oxidationsmittelzubereitung (B) als Oxidationsmittel Wasserstoffperoxid und/oder eines seiner festen Anlagerungsprodukte an organische oder anorganische Verbindungen, wie Harnstoff, Melamin sowie Natriumborat.

Bevorzugt beträgt die Menge an Oxidationsmittel im anwendungsbereiten Mittel 0,5 bis 12 Gew.-%, bevorzugt 2 bis 10 Gew.-% insbesondere bevorzugt zu 3 bis 6 Gew.-% (berechnet als 100 %-iges H₂O₂), jeweils bezogen auf das anwendungsbereite Mittel.

Solche Oxidationsmittelzubereitungen sind vorzugsweise wässrige, fließfähige Oxidationsmittelzubereitungen. Dabei sind bevorzugte Zubereitungen dadurch gekennzeichnet, dass die fließfähige Oxidationsmittelzubereitung - bezogen auf ihr Gewicht - 40 bis 95 Gew.-%, vorzugsweise 50 bis 90 Gew.-%, besonders bevorzugt 55 bis 85 Gew.-%, weiter bevorzugt 60 bis 80 Gew.-% und insbesondere 65 bis 75 Gew.-% Wasser enthält.

Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z. B. bestimmte Enzyme, lodide, Chinone oder Metallionen.

Weiterhin hat es sich als vorteilhaft erweisen, wenn die Oxidationsmittelzubereitungen mindestens einen Stabilisator oder Komplexbildner enthalten. Besonders bevorzugte Stabilisatoren sind Phenacetin, Alkalibenzoate (Natriumbenzoat) und Salicylsäure.

Erfindungsgemäß bevorzugt ist auch der Einsatz von sogenannten Komplexbildnern. Komplexbilder sind Stoffe, die Metallionen komplexieren können. Bevorzugte Komplexbildner sind sogenannte Chelatkomplexbildner, also Stoffe, die mit Metallionen cyclische Verbindungen bilden, wobei ein einzelner Ligand mehr als eine Koordinationsstelle an einem Zentralatom besetzt, d. h. mindestens "zweizähnig" ist. Die Zahl der gebundenen Liganden hängt von der Koordinationszahl des zentralen Ions ab.

Erfindungsgemäß bevorzugte Komplexbildner sind stickstoffhaltigen Polycarbonsäuren, insbesondere EDTA, und Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Die Färbezubereitung und gegebenenfalls Oxidationsmittelzubereitung enthalten weitere Hilfs- und Zusatzstoffe. So hat es sich erfindungsgemäß als bevorzugt erwiesen, wenn die Färbezubereitung und/oder die Oxidationsmittelzubereitung mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Zur weiteren Steigerung der Leistung der Oxidationsmittelzubereitung können der erfindungsgemäßen Zusammensetzung zusätzlich mindestens eine gegebenenfalls hydratisierte SiO₂-Verbindung zugesetzt. Es kann erfindungsgemäß bevorzugt sein, die gegebenenfalls hydratisierten SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der SiO₂-Verbindungen (ohne deren Wasseranteil) in den Mitteln wieder.

Hinsichtlich der gegebenenfalls hydratisierten SiO₂-Verbindungen unterliegt die vorliegende Erfindung prinzipiell keinen Beschränkungen. Bevorzugt sind Kieselsäuren, deren Oligomeren und Polymeren sowie deren Salze. Bevorzugte Salze sind die Alkalisalze, insbesondere die Kalium und Natriumsalze. Die Natriumsalze sind ganz besonders bevorzugt. Erfindungsgemäß ganz besonders bevorzugt sind Wassergläser, die aus einem Silikat der Formel (SiO₂)ₙ(Na₂O)ₘ(K₂O)ₚ gebildet werden, wobei n steht für eine positive rationale Zahl und m und p stehen unabhängig voneinander für eine positive rationale Zahl oder für 0, mit den Maßgaben, dass mindestens einer der Parameter m oder p von 0 verschieden ist und das Verhältnis zwischen n und der Summe aus m und p zwischen 1:4 und 4:1 liegt. Neben den durch die Summenformel beschriebenen Komponenten können die Wassergläser in geringen Mengen noch weitere Zusatzstoffe, wie beispielsweise Phosphate oder Magnesiumsalze, enthalten.

Erfindungsgemäß besonders bevorzugte Wassergläser werden unter anderem von der Firma Henkel unter den Bezeichnungen Ferrosil 119, Natronwasserglas 40/42, Portil A, Portil AW und Portil W und von der Firma Akzo unter der Bezeichnung Britesil C20 vertrieben.

Vorzugsweise sind die Zubereitung (C) und/oder gegebenenfalls die Oxidationsmittelzubereitung (B) als fließfähigen Zubereitungen konfektioniert.

Vorzugsweise wird den fließfähigen Zubereitungen (B) und/oder (C) weiterhin ein Emulgator bzw. ein Tensid zugesetzt, wobei oberflächenaktive Substanzen je nach Anwendungsgebiet als Tenside oder als Emulgatoren bezeichnet werden und aus anionischen, kationischen, zwitterionischen, amphoteren und nichtionischen Tensiden und Emulgatoren ausgewählt sind.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Bevorzugte anionische Tenside sind Alkylsulfate, Alkylethersulfate und Ethercarbonsäuren mit 10 bis 18 C-Atomen in der Alkylgruppe und bis zu 12 Glykolethergruppen im Molekül.

Als zwitterionische Tenside werden solche oberflächenaktiven Verbindungen bezeichnet, die im Molekül mindestens eine quartäre Ammoniumgruppe und mindestens eine Carboxylat-, Sulfonat- oder Sulfat-Gruppe tragen. Besonders geeignete zwitterionische Tenside sind die sogenannten Betaine wie die N-Alkyl-N,N-dimethylammoniumglycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxy-ethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter amphoteren Tensiden werden solche oberflächenaktiven Verbindungen verstanden, die außer einer C₈-C₂₄-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylg-lycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte amphotere Tenside sind das N-Kokosalkylaminopropionat, das Kokosacylaminoethylaminopropionat und das C₁₂-C₁₈-Acylsarcosin.

Weiterhin hat es sich als vorteilhaft erwiesen, wenn die erfindungsgemäßen Färbemittel weitere, nichtionogene grenzflächenaktive Stoffe, enthalten. Nichtionische Tenside enthalten als hydrophile Gruppe z. B. eine Polyolgruppe, eine Polyalkylenglykolethergruppe oder eine Kombination aus Polyol- und Polyglykolethergruppe. Als nichtionische Tenside eignen sich insbesondere C₈-C₂₂-Alkylmono- und oligoglycoside und deren ethoxylierte Analoga. Insbesondere die nichtethoxylierten Verbindungen haben sich als besonders geeignet erwiesen. Als weitere bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die anionischen, nichtionischen, zwitterionischen oder amphoteren Tenside werden in Mengen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugt sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethyl-ammoniumchloride, z. B. Cetyltrimethylammoniumchlorid, Stearyltrimethylammoniumchlorid, Di-stearyldimethylammoniumchlorid, Lauryldimethylammoniumchlorid, Lauryldimethylbenzylammo-niumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Weitere erfindungsgemäß verwendbare kationische Tenside stellen die quaternisierten Proteinhydrolysate dar.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid S 18 im Handel erhältliche Stearamidopropyl-dimethylamin dar.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex, Dehyquart und Armocare vertrieben. Die Produkte Armocare VGH-70, ein N,N-Bis(2-Palmitoyloxyethyl)dimethylammoniumchlorid, sowie Dehyquart F-75, Dehyquart C-4046, Dehyquart L80 und Dehyquart AU-35 sind Beispiele für solche Esterquats.

Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

In einer bevorzugten Ausführungsform können nicht-ionische, zwitterionische und/oder amphotere Tenside sowie deren Mischungen bevorzugt sein.

Eine erfindungsgemäß bevorzugte Ausführungsform der vorliegenden Erfindung besteht darin, dass das anwendungsbereite Mittel einen pH-Wert zwischen 7 und 12, insbesondere zwischen 8 und 11,5, insbesondere bevorzugt zwischen 8,5 und 11,5, besitzt. Bei den pH-Werten im Sinne der vorliegenden Erfindung handelt es sich um pH-Werte, die bei einer Temperatur von 22 °C gemessen wurden.

Üblicherweise wird der pH-Wert mit pH-Stellmitteln eingestellt. Zur Einstellung des pH-Werts sind dem Fachmann in der Kosmetik gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren.

Erfindungsgemäß einsetzbare, organische Alkalisierungsmittel werden bevorzugt ausgewählt aus Alkanolaminen aus primären, sekundären oder tertiären Aminen mit einem C₂-C₆-Alkylgrund-körper, der mindestens eine Hydroxylgruppe trägt. Bevorzugte Alkanolamine sind Monoethanolamin und Triethanolamin. Es hat sich aber im Rahmen der Untersuchungen zur vorliegenden Erfindung herausgestellt, dass weiterhin erfindungsgemäß bevorzugte Mittel dadurch gekennzeichnet sind, dass sie zusätzlich ein anorganisches Alkalisierungsmittel enthalten. Das erfindungsgemäße, anorganische Alkalisierungsmittel wird bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat. Ganz besonders bevorzugt sind Natriumhydroxid und/oder Kaliumhydroxid. Die als erfindungsgemäßes Alkalisierungsmittel einsetzbaren basischen Aminosäuren werden bevorzugt ausgewählt aus der Gruppe, die gebildet wird aus L-Arginin, D-Arginin, D/L-Arginin, L-Lysin, D-Lysin, D/L-Lysin, besonders bevorzugt L-Arginin, D-Arginin, D/L-Arginin als ein Alkalisierungsmittel im Sinne der Erfindung eingesetzt. Schließlich ist ein weiteres bevorzugtes Alkalisierungsmittel Ammoniak.

Bevorzugt werden die Alkalisierungsmittel in einer Menge von 0,05 bis 10 Gew.-%, insbesondere von 0,5 bis 5 Gew.-%, jeweils bezogen auf das Gesamtgewicht des anwendungsbereiten Mittels, enthalten.

Ferner können die erfindungsgemäßen Mittel weitere Wirk-, Hilfs- und Zusatzstoffe enthalten, beispielsweise nichtionische Polymere (wie Vinylpyrrolidinon/Vinylacrylat-Copolymere, Polyvinylpyrrolidinon und Vinylpyrrolidinon/Vinylacetat-Copolymere, Polyethylenglykole und Polysiloxane); kationische Polymere (wie quaternisierte Celluloseether, Polysiloxane mit quaternären Gruppen, Dimethyldiallylammoniumchlorid-Polymere, Acrylamid-Dimethyldiallyl-ammonium-chlorid-Copolymere, mit Diethylsulfat quaternierte Dimethylamino-ethylmethacrylat-Vinyl-pyrrolidinon-Copolymere, Vinylpyrrolidinon-Imidazolinium-methochlorid-Copolymere und quaternierter Polyvinylalkohol); zwitterionische und amphotere Polymere (wie Acrylamidopropyl-trimethylammoniumchlorid/Acrylat-Copolymere und Octylacrylamid/Methyl-methacrylat/t-Butyl-aminoethylmethacrylat/2-Hydroxypropylmethacrylat-Copolymere); anionische Polymere (wie Polyacrylsäuren, vernetzte Polyacrylsäuren, Vinylacetat/Crotonsäure-Copolymere, Vinylpyrrolidin-on/Vinylacrylat-Copolymere, Vinylacetat/ Butylmaleat/Isobornylacrylat-Copolymere, Methylvinyl-ether/Malein-säureanhydrid-Copolymere und Acrylsäure/Ethylacrylat/N-t-Butyl-acrylamid-Terpolymere); weitere Verdickungsmittel (wie Agar-Agar, Guar-Gum, Alginate, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol); Strukturanten (wie Glucose, Maleinsäure und Milchsäure); haarkonditionierende Verbindungen (wie Phospholipide, Sojalecithin, Ei-Lecitin und Kephaline sowie Silikonöle); Proteinhydrolysate (insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate); faserstrukturverbessernde Wirkstoffe (insbesondere Mono-, Di- und Oligosaccharide wie beispielsweise Glucose, Galactose, Fructose, Fruchtzucker und Lactose); Entschäumer (wie Silikone, bevorzugt Dimethicon); Farbstoffe zum Anfärben des Mittels; Antischuppenwirkstoffe (wie Piroctone Olamine, Zink Omadine und Climbazol); Lichtschutzmittel beziehungsweise UV-Blocker (insbesondere derivatisierte Benzophenone, Zimtsäure-Derivate und Triazine); Wirkstoffe (wie Panthenol, Pantothensäure, Pantolacton, Allantoin, Pyrrolidinoncarbonsäuren und deren Salze sowie Bisabolol); Vitamine, Provitamine und Vitaminvorstufen (insbesondere solche der Gruppen A, B₃, B₅, B₆, C, E, F und H); Cholesterin; Konsistenzgeber (wie Zuckerester, Polyolester oder Polyolalkylether); Fette und Wachse (wie Bienenwachs, Montanwachs und Paraffine); Fettsäurealkanolamide; Quell- und Penetrationsstoffe (wie Glycerin, Propylenglykolmonoethylether, Carbonate, Hydrogencarbonate, Guanidine, Harnstoffe sowie primäre, sekundäre und tertiäre Phosphate); Trübungsmittel (wie Latex, Styrol/PVP- und Styrol/Acrylamid-Copolymere); Perlglanzmittel (wie Ethylenglykolmono-und -distearat sowie PEG-3-distearat); Pigmente; Parfümöle; Treibmittel (wie Propan-Butan-Gemische, N₂O, Dimethylether, CO₂ und Luft) sowie Antioxidantien.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die erfindungsgemäßen Mittel eignen sich insbesondere dazu, eine einheitliche und gleichmäßige Färbung zu erzielen.

Ein weiterer Gegenstand der vorliegenden Erfindung ist daher die Verwendung eines Mittels, enthaltend mindestens einen partikelförmigen Bestandteil, der einen Partikelkern, enthaltend mindestens ein Oxidationsfarbstoffvorprodukt, umfasst und eine diesen Kern umgebende Hülle aufweist, wobei die Hülle mindestens ein Verkapselungsmaterial, ausgewählt unter
a) Homo- und/oder Copolymeren von Methacrylsäure und/oder Methacrylsäureestern (Methacrylaten) und/oder
b) Homo- und/oder Copolymeren von Vinylacetat und/oder
c) Schellack
und mindestens ein Trennmittel, ausgewählt aus Partialglyceriden von Fettsäuren, Metallseifen und anorganischen, pulverförmigen Trennmitteln, ausgewählt aus Graphit, Talk und Glimmer, enthält,
zur Verbesserung der Gleichmäßigkeit der Färbung von keratinischen Fasern, insbesondere menschlicher Haare.

Bezüglich bevorzugter Ausführungsformen der erfindungsgemäßen Verwendung gilt hinsichtlich der Stoffauswahl mutatis mutandis das zu den erfindungsgemäßen Mitteln Gesagte.

Ein weiterer Gegenstand der vorliegenden Erfindung ist ein Verfahren zur Herstellung von Mitteln zum Färben von keratinischen Fasern, das in einem ersten Schritt das Beschichten mindestens eines Partikelkerns, enthaltend mindestens ein Oxidationsfarbstoffvorprodukt, mit einem Beschichtungsmittel unter Ausbildung einer Hülle, die aus mindestens einem
a) Homo- und/oder Copolymeren von Methacrylsäure und/oder Methacrylsäureestern und/oder
b) Homo- und/oder Copolymeren von Vinylacetat und/oder
c) Schellack
sowie aus mindestens einem Trennmittel, ausgewählt aus Partialglyceriden von Fettsäuren, Metallseifen und anorganischen, pulverförmigen Trennmitteln, ausgewählt aus Graphit, Talk und Glimmer, besteht,
und in einem zweiten Schritt das Abmischen des/der beschichteten Teilchen mit einer wässrigen Oxidationszubereitung, enthaltend Wasserstoffperoxid, umfasst, wobei das Beschichten der Partikelkerne zweistufig durchgeführt wird, wobei die zu beschichtenden Partikelkerne in einer Wirbelschichtapparatur in einer ersten Stufe gegebenenfalls wiederholt mit einer Lösung oder Dispersion, enthaltend mindestens
a) ein Homo- und/oder Copolymer von Methacrylsäure und/oder Methacrylsäureestern und/oder
b) ein Homo- und/oder Copolymer von Vinylacetat und/oder
c) Schellack
und mindestens ein Trennmittel, ausgewählt aus Partialglyceriden von Fettsäuren, Metallseifen und anorganischen, pulverförmigen Trennmitteln, ausgewählt aus Graphit, Talk und Glimmer, besprüht und getrocknet werden,
und in einer zweiten Stufe mit einer wässrigen Dispersion, enthaltend mindestens Siliciumdioxid, besprüht und getrocknet werden.

Im ersten Schritt des erfindungsgemäßen Verfahrens wird mindestens ein partikelförmiger Inhaltsstoff mit mindestens einem der vorstehend genannten Stoffe beschichtet. Dieser Schritt lässt sich in den unterschiedlichsten Apparaturen problemlos durchführen.

Alternativ zum Einsatz eines Mischergranulators kann auch eine Wirbelschichtapparatur zur Ausbildung des Feststoffcoatings genutzt werden. Erfindungsgemäße Verfahren, bei denen die Beschichtung der zu beschichtenden Partikel in einer Wirbelschichtapparatur durchgeführt wird, sind bevorzugt.

Auch hier kann gleichzeitig Flüssigkeit auf die Körner aufgebracht werden. Die Beschichtung kann dabei gleichzeitig mit der Trocknung erfolgen (beispielsweise in einer Wirbelschichtapparatur, in der die Granulate mit einer Lösung oder Dispersion mindestens eines der oben genannten Stoffe beaufschlagt und gleichzeitig getrocknet werden), es ist aber auch möglich und bevorzugt, die Trocknung nach der Beschichtung, also zeitlich anschließend an diese, durchzuführen.

Es kann eine dichte Hülle erzeugt werden, indem eine Lösung oder Dispersion des/der oben genannten Stoffe(s) auf die zu beschichtenden Partikel aufgebracht wird. Diese Lösung bzw. Dispersion kann auch weitere filmbildende Substanzen enthalten.

Alternative Beschichtungsverfahren, die sich zur Herstellung erfindungsgemäßer Zusammensetzungen eignen, sind das Strahlschichtcoating sowie Schmelzextrusionsverfahren.

Es hat sich als besonders effektiv zur Verhinderung eines Verklebens oder Verbackens der umhüllten Partikel herausgestellt, wenn die Partikel an der Oberfläche als zusätzliches Trennmittel Siliciumdioxid enthalten. Dazu werden die Partikel, welche von einer oder mehreren, gegebenenfalls in Dicke und Zusammensetzung verschiedenen Verkapselungsschichten umhüllt sind, mit einer wässrigen Dispersion von Siliciumdioxid besprüht und getrocknet.

## Patentansprüche

1. Mittel zum Färben von keratinischen Fasern, insbesondere menschlichen Haaren, enthaltend mindestens einen partikelförmigen Bestandteil, der einen Partikelkern, enthaltend mindestens ein Oxidationsfarbstoffvorprodukt, umfasst und eine diesen Kern umgebende Hülle aufweist, **dadurch gekennzeichnet, dass** die Hülle aus mindestens einem Verkapselungsmaterial, ausgewählt unter
a) Homo- und/oder Copolymeren von Methacrylsäure und/oder Methacrylsäureestern (Methacrylaten) und/oder
b) Homo- und/oder Copolymeren von Vinylacetat und/oder
c) Schellack
und mindestens einem Trennmittel, ausgewählt aus der Gruppe, die gebildet wird aus Partialglyceriden von Fettsäuren, Metallseifen und anorganischen, pulverförmigen Trennmitteln, ausgewählt aus Graphit, Talk und Glimmer,
besteht.

2. Mittel nach Anspruch 1, **dadurch gekennzeichnet, dass** die Hülle vorzugsweise zu mindestens 50 Gew.-%, weiter bevorzugt zu mindestens 70 Gew.-% und insbesondere zu mindestens 80 Gew.-% ihres Gewichts aus
a) Homo- und/oder Copolymeren von Methacrylsäure und/oder Methacrylsäureestern und/oder
b) Homo- und/oder Copolymeren von Vinylacetat und/oder
c) Schellack
besteht.

3. Mittel nach einem der Ansprüche 1 oder 2, **dadurch gekennzeichnet, dass** die Hülle Polyvinylacetat enthält.

4. Mittel nach einem der Ansprüche 1 oder 2, **dadurch gekennzeichnet, dass** die Hülle mindestens ein Copolymer aus Methacrylsäure, Methylacrylat und Methylmethacrylat enthält.

5. Mittel nach einem der Ansprüche 1 oder 2, **dadurch gekennzeichnet, dass** die Hülle mindestens ein Copolymer aus Methacrylsäure und Ethylacrylat enthält.

6. Mittel nach einem der Ansprüche 1 oder 2, **dadurch gekennzeichnet, dass** die Hülle mindestens ein Copolymer aus (2-Trimethylammonioethyl)methacrylatchlorid und mindestens einem zusätzlichen Monomer enthält, welches ausgewählt wird aus der Gruppe, die gebildet wird aus Ethylmethacrylat, Methylmethacrylat, Ethylacrylat und Methylacrylat.

7. Mittel nach einem der Ansprüche 1 oder 2, **dadurch gekennzeichnet, dass** die Hülle mindestens ein Copolymer aus Methylmethacrylat und Ethylacrylat enthält.

8. Mittel nach einem der Ansprüche 1 bis 7, **dadurch gekennzeichnet, dass** die Hülle als Trennmittel Talk enthält.

9. Mittel nach einem der Ansprüche 1 bis 8, **dadurch gekennzeichnet, dass** die partikelförmigen Bestandteile als zusätzliches Trennmittel Siliciumdioxid enthalten.

10. Mittel nach Anspruch 9, **dadurch gekennzeichnet, dass** die partikelförmigen Bestandteile, enthaltend als zusätzliches Trennmittel Siliciumdioxid, dadurch hergestellt werden, dass die umhüllten, partikelförmigen Bestandteile mit einer wässrigen Dispersion, enthaltend Siliciumdioxid, besprüht werden und anschließend getrocknet werden.

11. Mittel nach einem der Ansprüche 1 bis 10, **dadurch gekennzeichnet, dass** die den Partikelkern umgebende Hülle zusätzlich mindestens einen Porenbildner enthält.

12. Verwendung eines Mittels, enthaltend mindestens einen partikelförmigen Bestandteil, der einen Partikelkern, enthaltend mindestens ein Oxidationsfarbstoffvorprodukt, umfasst und eine diesen Kern umgebende Hülle aufweist, wobei die Hülle mindestens ein Verkapselungsmaterial, ausgewählt unter
a) Homo- und/oder Copolymeren von Methacrylsäure und/oder Methacrylsäureestern (Methacrylaten) und/oder
b) Homo- und/oder Copolymeren von Vinylacetat und/oder
c) Schellack,
und mindestens ein Trennmittel, ausgewählt aus der Gruppe, die gebildet wird aus Partialglyceriden von Fettsäuren, Metallseifen und anorganischen, pulverförmigen Trennmitteln, ausgewählt aus Graphit, Talk und Glimmer, enthält,
zur Verbesserung der Gleichmäßigkeit der Färbung von keratinischen Fasern, insbesondere menschlicher Haare.

13. Verfahren zur Herstellung von Mitteln zum Färben von keratinischen Fasern, das in einem ersten Schritt das Beschichten mindestens eines Partikelkerns, enthaltend mindestens ein Oxidationsfarbstoffvorprodukt, mit einem Beschichtungsmittel unter Ausbildung einer Hülle, die aus mindestens einem
a) Homo- und/oder Copolymeren von Methacrylsäure und/oder Methacrylsäureestern und/oder
b) Homo- und/oder Copolymeren von Vinylacetat und/oder
c) Schellack
sowie aus mindestens einem Trennmittel, ausgewählt aus Partialglyceriden von Fettsäuren,
Metallseifen und anorganischen, pulverförmigen Trennmitteln, ausgewählt aus Graphit, Talk und Glimmer, besteht,
und in einem zweiten Schritt das Abmischen des/der beschichteten Teilchen mit einer wässrigen Oxidationszubereitung, enthaltend Wasserstoffperoxid, umfasst,
**dadurch gekennzeichnet, dass** das Beschichten der Partikelkerne zweistufig durchgeführt wird, wobei die zu beschichtenden Partikelkerne in einer Wirbelschichtapparatur in einer ersten Stufe gegebenenfalls wiederholt mit einer Lösung oder Dispersion, enthaltend mindestens
a) ein Homo- und/oder Copolymer von Methacrylsäure und/oder Methacrylsäureestern und/oder
b) ein Homo- und/oder Copolymer von Vinylacetat und/oder
c) Schellack
und mindestens ein Trennmittel, ausgewählt aus Partialglyceriden von Fettsäuren, Metallseifen und anorganischen, pulverförmigen Trennmitteln, ausgewählt aus Graphit, Talk und Glimmer, besprüht und getrocknet werden,
und in einer zweiten Stufe mit einer wässrigen Dispersion, enthaltend mindestens Siliciumdioxid,
besprüht und getrocknet werden.

## Claims

1. An agent for dyeing keratinous fibers, in particular human hairs, containing at least one component in particle form, which component comprises a particle core, containing at least one oxidation dye precursor, and a shell surrounding this core, **characterized in that** the shell consists of at least one encapsulating material selected from
a) homopolymers and/or copolymers of methacrylic acid and/or methacrylic acid esters (methacrylates), and/or
b) homopolymers and/or copolymers of vinyl acetate, and/or
c) shellac,
and at least one release agent selected from the group formed by partial glycerides of fatty acids, metallic soaps, and inorganic, powdered release agents selected from graphite, talc and mica.

2. The agent according to claim 1, **characterized in that** at least 50 wt.%, more preferably at least 70 wt.% and in particular at least 80 wt.% of the weight of the shell consists of
a) homopolymers and/or copolymers of methacrylic acid and/or methacrylic acid esters, and/or
b) homopolymers and/or copolymers of vinyl acetate, and/or
c) shellac.

3. The agent according to claim 1 or 2, **characterized in that** the shell contains polyvinyl acetate.

4. The agent according to claim 1 or 2, **characterized in that** the shell contains at least one copolymer from methacrylic acid, methacrylate, and methyl methacrylate.

5. The agent according to claim 1 or 2, **characterized in that** the shell contains at least one copolymer from methacrylic acid and ethyl acrylate.

6. The agent according to claim 1 or 2, **characterized in that** the shell contains at least one copolymer from (2-trimethylammonio ethyl)methacrylate chloride and at least one additional monomer which is selected from the group formed by ethyl methacrylate, methyl methacrylate, ethyl acrylate and methacrylate.

7. The agent according to claim 1 or 2, **characterized in that** the shell contains at least one copolymer from methyl methacrylate and ethyl acrylate.

8. The agent according to one of claims 1 to 7, **characterized in that** the shell contains talc as the release agent.

9. The agent according to one of claims 1 to 8, **characterized in that** the components in particle form contain silicon dioxide as an additional release agent.

10. The agent according to claim 9, **characterized in that** the components in particle form, containing silicon dioxide as an additional release agent, are produced by spraying the encased components in particle form with an aqueous dispersion containing silicon dioxide, and then drying them.

11. The agent according to one of claims 1 to 10, **characterized in that** the shell surrounding the particle core additionally contains at least one pore former.

12. The use of an agent containing at least one component in particle form, which component comprises a particle core containing at least one oxidation dye precursor, and a shell surrounding this core, wherein the shell contains at least one encapsulating material, selected from
a) homopolymers and/or copolymers of methacrylic acid and/or methacrylic acid esters (methacrylates), and/or
b) homopolymers and/or copolymers of vinyl acetate, and/or
c) shellac,
and at least one release agent selected from the group formed by partial glycerides of fatty acids, metallic soaps, and inorganic, powdered release agents selected from graphite, talc and mica,
for improving the uniformity of the dyeing of keratinous fibers, in particular human hair.

13. A method for producing agents for dyeing keratinous fibers, which method comprises, in the first step, coating at least one particle core containing at least one oxidation dye precursor, with a coating agent in order to form a shell that consists of at least one
a) homopolymer and/or copolymer of methacrylic acid and/or methacrylic acid esters, and/or
b) homopolymers and/or copolymers of vinyl acetate, and/or
c) shellac,
and of at least one release agent, selected from partial glycerides of fatty acids, metallic soaps, and inorganic, powdered release agents, selected from graphite, talc and mica,
and comprises, in a second step, mixing the coated particle(s) with an aqueous oxidizing preparation, containing hydrogen peroxide,
**characterized in that** the particle cores are coated in two stages, the particle cores to be coated being sprayed, in a first stage, optionally repeatedly, in a fluidized bed apparatus with a solution or dispersion containing at least
a) one homopolymer and/or copolymer of methacrylic acid and/or methacrylic acid esters, and/or
b) one homopolymer and/or copolymer of vinyl acetate, and/or
c) shellac,
and at least one release agent selected from partial glycerides of fatty acids, metallic soaps, and inorganic, powdered release agents, selected from graphite, talc and mica, and being dried,
and being sprayed, in a second stage, with an aqueous dispersion containing at least silicon dioxide, and being dried.

## Revendications

1. Agent de coloration de fibres de kératine, en particulier de cheveux humains, contenant au moins un composant particulaire qui comporte un noyau de particules, contenant au moins un précurseur de colorant d'oxydation, et une enveloppe entourant ledit noyau, **caractérisé en ce que** l'enveloppe est constituée d'au moins une matière d'encapsulation choisie parmi
a) les homopolymères et/ou copolymères de l'acide méthacrylique et/ou des esters de l'acide méthacrylique (méthacrylate) et/ou
b) les homopolymères et/ou copolymères de l'acétate de vinyle et/ou
c) la gomme-laque
et au moins un agent de séparation choisi dans le groupe qui est constitué des glycérides partiels d'acides gras, des savons métalliques et des agents de séparation minéraux pulvérulents choisis parmi le graphite, le talc et le mica.

2. Agent selon la revendication 1, **caractérisé en ce que** l'enveloppe est constituée, de préférence d'au moins 50% en poids, plus préférablement d'au moins 70% en poids et en particulier d'au moins 80% en poids,
a) d'homopolymères et/ou de copolymères de l'acide méthacrylique et/ou des esters de l'acide méthacrylique et/ou
b) d'homopolymères et/ou de copolymères de l'acétate de vinyle et/ou
c) de gomme-laque.

3. Agent selon l'une des revendications 1 ou 2, **caractérisé en ce que** l'enveloppe contient de l'acétate de polyvinyle.

4. Agent selon l'une des revendications 1 ou 2, **caractérisé en ce que** l'enveloppe contient au moins un copolymère de l'acide méthacrylique, de l'acrylate de méthyle et du méthacrylate de méthyle.

5. Agent selon l'une des revendications 1 ou 2, **caractérisé en ce que** l'enveloppe contient au moins un copolymère de l'acide méthacrylique et de l'acrylate d'éthyle.

6. Agent selon l'une des revendications 1 ou 2, **caractérisé en ce que** l'enveloppe contient au moins un copolymère du chlorure de (2-triméthylammonioéthyle)-méthacrylate et au moins un monomère supplémentaire qui est choisi dans le groupe qui est formé par le méthacrylate d'éthyle, le méthacrylate de méthyle, l'acrylate d'éthyle et l'acrylate de méthyle.

7. Agent selon l'une des revendications 1 ou 2, **caractérisé en ce que** l'enveloppe contient au moins un copolymère de méthacrylate de méthyle et d'acrylate d'éthyle.

8. Agent selon l'une des revendications 1 à 7, **caractérisé en ce que** l'enveloppe contient comme agent de séparation du talc.

9. Agent selon l'une des revendications 1 à 8, **caractérisé en ce que** les composants particulaires contiennent comme agent de séparation supplémentaire du dioxyde de silicium.

10. Agent selon la revendication 9, **caractérisé en ce que** les composants particulaires contenant comme agent de séparation supplémentaire du dioxyde de silicium sont produits par aspersion des composants particulaires enrobés avec une dispersion aqueuse contenant du dioxyde de silicium puis par séchage.

11. Agent selon l'une des revendications 1 à 10, **caractérisé en ce que** l'enveloppe entourant le noyau de particules contient en outre au moins un agent porogène.

12. Utilisation d'un agent contenant au moins un composant particulaire qui comporte un noyau de particules, contenant au moins un précurseur de colorant d'oxydation, et une enveloppe entourant ledit noyau, l'enveloppe étant constituée d'au moins une matière d'encapsulation choisie parmi
a) les homopolymères et/ou copolymères de l'acide méthacrylique et/ou des esters de l'acide méthacrylique (méthacrylate) et/ou
b) les homopolymères et/ou copolymères de l'acétate de vinyle et/ou
c) la gomme-laque
et au moins un agent de séparation choisi dans le groupe qui est constitué des glycérides partiels d'acides gras, des savons métalliques et des agents de séparation minéraux pulvérulents choisis parmi le graphite, le talc et le mica,
pour améliorer l'uniformité de la coloration des fibres de kératine, en particulier de cheveux humains.

13. Procédé de préparation d'agents de coloration de fibres de kératine, qui comprend dans une première étape le revêtement d'au moins un noyau de particules, contenant au moins un précurseur de colorant d'oxydation, avec un agent de revêtement pour former une enveloppe qui est constituée d'au moins
a) un homopolymère et/ou copolymère de l'acide méthacrylique et/ou des esters de l'acide méthacrylique (méthacrylate) et/ou
b) un homopolymère et/ou copolymère de l'acétate de vinyle et/ou
c) la gomme-laque
et au moins un agent de séparation choisi dans le groupe qui est constitué des glycérides partiels d'acides gras, des savons métalliques et des agents de séparation minéraux pulvérulents choisis parmi le graphite, le talc et le mica, et
dans une seconde étape le mélange de la ou des particules revêtues avec une préparation d'oxydation aqueuse, contenant du peroxyde d'hydrogène,
**caractérisé en ce que** le revêtement des noyaux de particules est effectué en deux étapes dans lesquelles, dans un appareil à lit fluidisés, les noyaux de particules à revêtir sont aspergés dans une première étape, éventuellement répétée, avec une solution ou une dispersion contenant au moins
a) un homopolymère et/ou copolymère de l'acide méthacrylique et/ou des esters de l'acide méthacrylique (méthacrylate) et/ou
b) un homopolymère et/ou copolymère de l'acétate de vinyle et/ou
c) la gomme-laque
et au moins un agent de séparation choisi dans le groupe qui est constitué des glycérides partiels d'acides gras, des savons métalliques et des agents de séparation minéraux pulvérulents choisis parmi le graphite, le talc et le mica,
puis séchés,
et, dans une seconde étape, sont aspergés avec une dispersion aqueuse, contenant au moins du dioxyde de silicium, puis séchés.
